Europäisches Patentamt

European Patent Office

Office européen des brevets

(11) Publication number: **0 429 059 A2**

# EUROPEAN PATENT APPLICATION

(21) Application number: 90122178.8

(51) Int. Cl.⁵: **C07C 23/02, C07C 17/02**

(22) Date of filing: 20.11.90

(30) Priority: 21.11.89 JP 300818/89
26.12.89 JP 334919/89
29.10.90 JP 288452/90
29.10.90 JP 288453/90

(43) Date of publication of application:
29.05.91 Bulletin 91/22

(84) Designated Contracting States:
DE FR GB IT

(71) Applicant: TOSOH CORPORATION
4560, Kaisei-cho
Shinnanyo-shi Yamaguchi-ken(JP)

(72) Inventor: Takao, Matsuba
3-4-17, Madokoro
Shinnanyo-shi, Yamaguchi-ken(JP)
Inventor: Masashige, Kubo
4-16-2-1105, Chuo-Honmachi
Adachi-ku, Tokyo(JP)
Inventor: Kouji, Kawabata
1-14, Nishimasu-cho
Shinnanyo-shi, Yamaguchi-ken(JP)

(74) Representative: Vossius & Partner
Siebertstrasse 4 P.O. Box 86 07 67
W-8000 München 86(DE)

(54) Process for producing hexabromocyclododecane.

(57) Process for producing thermostable γ-HBCD selectively by reacting bromine with CDT in the presence of an organic solvent, an alcohol of 1 - 4 carbons or an organic solvent containing the alcohol as a reaction solvent. CDT is dropwisely added to the bromine solution, and the concentration of CDT substrate is in the range of from 0.1 to 20 weight/volume % relative to the reaction solvent.

EP 0 429 059 A2

# PROCESS FOR PRODUCING HEXABROMOCYCLODODECANE

The present invention relates to a process for producing 1,2,5,6,9,10-hexabromocyclododecane which has excellent heat resistance and is useful as a fire retardant of polymers.

1,2,5,6,9,10-hexabromocyclododecane (hereinafter referred to as HBCD) is a well known fire retardant, and is used for polystyrene and other resins. This fire retardant is synthesized by an addition reaction of bromine to 1,5,9-cis,trans,trans-cyclododecatriene (hereinafter referred to as CDT).

German Patent 1,147,574 describes a bromine addition reaction employing ethyl alcohol as the reaction solvent and being conducted by dropping bromine to a solution of CDT in ethyl alcohol.

Several mixed solvent systems are disclosed for similar processes. For example, JP-B-Sho-49-24474 (The term "JP-B" as used herein means an "examined Japanese patent publication".) discloses a mixed solvent system of an alcohol and benzene; JP-B-Sho-49-24475, a mixed solvent system of an alcohol and an ester; US Patent 3,833,675, a mixed solvent system of t-butyl alcohol and benzene; JP-B-Sho-50-5187, a mixed solvent system of an alcohol and a halogenated hydrocarbon; and European Patent 181,414, a mixed solvent system of an alcohol and dioxane.

Further, JP-B-Sho-53-12510 discloses a method in which a solvent is placed in a reactor, and thereto CDT and bromine are simultaneously added dropwise.

HBCD formed by the bromine addition to CDT is known to include three isomers having different physical properties (E.R. Larsen and E.L. Ecker: J. Fire Sci., 4, 261 (1986)). It is reported that, in high-performance liquid chromatography employing an ODS reversed phase column, the isomers named $\alpha$-HBCD, $\beta$-HBCD, and $\gamma$-HBCD are eluted out in this order. The inventors of the present invention isolated the respective isomers and measured the properties: the melting points of $\alpha$-, $\beta$-, and $\gamma$-isomers being respectively 184 - 186°C, 168 - 171°C, and 196 -198°C; and the temperatures at 5% heat loss in weight of the isomers being respectively 242°C, 217°C and 245°C, and the temperatures at 50% heat loss in weight of the isomers being respectively 255°C, 232°C and 258°C by thermogravimetric analysis (in the air, at temperature elevation rate of 10°C/min.). Accordingly, the heat stability descends in the order of $\gamma$-HBCD, $\alpha$-HBCD, and $\beta$-HBCD.

The HBCD used as a fire retardant is mainly composed of the $\gamma$-isomer. The quality of the HBCD is greatly affected by the ratio of the isomers contained therein. The higher content of $\beta$-HBCD, which has a low melting point and low heat-stability, brings down the melting point of the HBCD, and gives rise to disadvantages of causing thermal decomposition at a lower temperature to cause corrosion of a molding machine or discoloration of a resin at molding of the resin.

The method disclosed in German Patent 1,147,574 involves the problems that an insoluble resinous matter deposits from the solution during the reaction to make the stirring difficult and to render the scale-up of the reactor difficult, and further that the resulting HBCD is unsatisfactory in heat-resistance owing to the low melting point thereof.

When the reaction is conducted in an aforementioned mixed solvent system proposed to solve these problems, the deposition of the resinous matter during the reaction is avoided, but the problem of low heat resistance still remains unsolved because of the low melting point of the produced HBCD.

The method disclosed in JP-B-Sho-53-12510 also involves the problem of low heat resistance owing to the low melting point of the produced HBCD.

Furthermore, in the conventional reaction methods mentioned above, side reactions such as bromination at an allyl position of CDT, dehydrobromination of brominated CDT, and bromination of the solvent tend to occur besides the intended addition of bromine to the double bonds of CDT during the bromination of CDT, which causes decrease of the yield, contamination of HBCD crystal with impurity, and other problems. The impurity also causes corrosion of molding machines and coloration of resins.

Hence, selective production of thermostable $\gamma$-HBCD has been looked for.

The present invention intends to provide a process for producing thermostable $\gamma$-HBCD selectively.

The present invention provides a process for producing HBCD by reacting bromine with CDT in the presence of an organic solvent, the process comprising adding dropwise CDT to a solution of bromine in an alcohol of 1 - 4 carbons or in an organic solvent containing the alcohol.

The present invention also provides a process for producing HBCD by reacting bromine with CDT in the presence of an alcohol of 1 - 4 carbons or an organic solvent containing the alcohol as a reaction solvent, the concentration of the CDT substrate being in the range of from 0. 1 to 20 weight/volume % ([grams of CDT/mls of solvent] x 100) relative to the reaction solvent.

The inventors of the present invention studied comprehensively the methods for producing highly thermos table $\gamma$-HBCD selectively.

2

As the results, the reaction conducted by dropping CDT to a solution of bromine in an alcohol of 1 -4 carbons or in a solvent containing the alcohol has been found to improve remarkably the selectivity of γ-HBCD and to decrease remarkably the unknown matters considered to be formed by a side reaction other than the bromine addition reaction in comparison with conventional processes of dropping bromine to CDT dissolved in an organic solvent or of dropping CDT and bromine simultaneously to a reaction solvent preliminarily placed in a reactor.

Further, in the reaction of bromine with CDT in the presence of an alcohol of 1 - 4 carbons or a solvent containing the alcohol, the limitation of the concentration of the substrate CDT within the range of from 0.1 to 20 wt/vol % relative to the solvent has been found to give remarkably improved selectivity of γ-HBCD and remarkable decrease of unknown matters considered to be formed by a side reaction other than the bromine addition reaction in comparison with a reaction conducted at relatively high concentration of 22 to 58% of the substrate as in the aforementioned prior art.

The solvent employed in the present invention is an alcohol of 1 to 4 carbons or an organic solvent containing the alcohol. The alcohol of 1 to 4 carbons includes methanol, ethanol, n-propanol, isopropanol, n-butanol, sec-butanol, isobutanol, tert-butanol, ethylene glycol, diethylene glycol, propylene glycol, and the like. Among the alcohols particularly preferable are ethanol, n-propanol, tert-butanol, etc. The solvent to be mixed with the alcohol includes ethers, halogenated hydrocarbons, esters, and so forth. As the specific examples of the solvent, the ethers includes dipropyl ether, diisopropyl ether, tetrahydrofuran (THF), dioxane, diethylene glycol dimethyl ether, diethylene glycol diethyl ether, and the like; the halogenated hydrocarbons include carbon tetrachloride, chloroform, methylene chloride, ethylene dichloride (EDC), and the like; and the esters include ethyl acetate, methyl acetate, 2-methoxyethyl acetate, and the like. As the mixed solvent, particularly preferable in consideration of the reaction results are ethanol-ethyl acetate, ethanol-THF, ethanol-dioxane, ethanol-methylene chloride, ethanol-EDC, and the like.

The temperature for conducting the process of the present invention is not limited specially. However, an excessively high temperature for the reaction is not preferable, since substitution reactions other than the bromine addition tend to occur to result in increase of impurities or a reaction of the solvent with bromine tends to occur at a high temperature. On the contrary, an extremely low temperature for the reaction is also not preferable since the reaction velocity is low to render the reaction incomplete and to cease the reaction at an intermediate stage although the deterioration of the solvent is prevented. Accordingly the reaction is conducted usually within the range of from about $-20°$ C to about $50°$ C.

The reaction time for conducting the process of the present invention depends on the reaction temperature, the amount of the materials used, and so forth. Usually, the dropping addition of CDT is conducted for the time of from about 10 minutes to about 10 hours, and the reaction was further allowed to proceed for about 3 hours.

The amount of bromine relative to CDT is not less than 3.0, preferably in the range of from 3.0 to 10.0 in terms of $Br_2$/CDT (molar ratio). At the ratio of less than 3.0, bromine is insufficient relative to CDT to complete the reaction, while at the ratio of exceeding 10, the excess bromine undesirably tends to cause side reactions.

The concentration of CDT relative to the organic solvent for reaction is in the range of from 0.1 to 20 wt/vol %, preferably 0.5 to 10 wt/vol %. The reaction at a concentration of CDT lower than 0.1 wt/vol % will not improve the selectivity of γ-HBCD as expected in comparison with the reaction at a concentration of 0.1 wt/vol %, and is not advantageous also from the economical point of view. The reaction at a concentration of CDT higher than 20 wt/vol % remarkably decreases the selectivity of γ-HBCD, and is undesirable.

The reaction conducted at a CDT substrate concentration within the range of 0.1 to 20 wt/vol %, preferably 0.5 to 10 wt/vol % will improve the selectivity of γ-HBCD in comparison with the reaction conducted at a concentration thereof higher than 20 wt/vol % in any of the reaction methods of dropping bromine to a solution of CDT, dropping CDT to a solution of bromine, and dropping bromine and CDT simultaneously to a solvent when compared at an equal concentration of the substrate CDT.

When compared where the concentration of CDT substrate is equall to the solvent, the reaction procedure in which CDT is dropped in the solution of bromine shows a more improved selectivity of γ-HBCD than the reaction procedure where bromine is dropped in the solution of CDT or bromine and CDT are simultaneously dropped in the solvent.

In particular, the selectivity of γ-HBCD is greatly improved by conducting reaction by dropping CDT to a solution of bromine at a CDT substrate concentration of from 0.1 to 20 wt/vol %, preferably from 0.5 to 10 wt/vol % relative to the reaction solvent.

After completion of the reaction, the produced HBCD can be isolated as a powdery matter by a known method. For example, the deposited crystalline matter is collected by filtration as crystalline HBCD. The solvent recovered as filtrate may be repeatedly used after replenishing a fresh solvent. In another method, the reaction solution after completion of the reaction may be poured into a poor solvent to separate the crystalline matter.

The practice of the method of the present invention allows production of the $\gamma$-isomer of HBCD at a high selectivity, and greatly decreases formation of impurities coming from the CDT. Accordingly, HBCD which is superior in color and thermostability has come to be produced.

The present invention is described in more detail referring to Examples without limiting the invention in any way.

Examples 1 - 9

A reaction solvent having a composition shown in Table 1 or Table 2, and bromine were placed in a round-bottomed flask equipped with a reflux condenser and a stirrer. Thereto, CDT in an amount shown in Table 1 or Table 2 was added dropwise at 30°C in 2 hours to cause reaction. After completion of the addition of the CDT, the reaction mixture was aged further at 30°C for 2 hours. The resulting slurry after the completion of the reaction was analyzed by high-speed chromatography (column: TSK gel-ODS-80T made by Tosoh Corporation, eluent: acetonitrile/water = 80/20 vol ratio, wave length: UV 215 nm). The results of the analysis are shown collectively in Tables 1 and 2. The components which could not be identified are shown as "unknown". In the Tables, DBCD (dibromocyclododecadiene) and TBCD (tetrabromocyclododecene) are reaction intermediates in the HBCD production. The TBCD includes isomers, which were analyzed by high-performance liquid chromatography using an ODS reversed phase column, and were named $\alpha$-TBCD and B-TBCD respectively according to the order of elution from the chromatographic column.

The quantity and the rate of the yield of HBCD were calculated of the total of the HBCD isomers. The selectivity of $\gamma$-HBCD are represented by the ratio of the quantity of the yield of $\gamma$-HBCD to the total quantity of HBCD isomers ($\gamma$-HBCD / ($\alpha$-HBCD + $\beta$-HBCD + $\gamma$-HBCD)).

After the completion of the reaction, the reaction mixture was filtered, and the separated crystalline matter was dried. The melting point thereof was measured. The results are shown collectively in Tables 1 and 2.

Comparative Examples 1 - 3

A reaction solvent having a composition shown in Table 3, and CDT were placed in a round-bottomed flask equipped with a reflux condenser and a stirrer. Thereto, bromine in an amount shown in Table 3 was added dropwise at 30°C in 2 hours to cause reaction. After completion of the addition of the bromine, the reaction mixture was aged further at 30°C for 2 hours. After completion of the reaction, the post-treatment and the analysis were conducted in the same manner as in Examples 1 - 9. The results are summarized in Table 3.

Comparative Example 4

A reaction solvent having a composition shown in Table 3 was placed in a round-bottomed flask equipped with a reflux condenser and a stirrer. Thereto, bromine and CDT in amounts shown in Table 3 were simultaneously added dropwise at 30°C in 2 hours to cause reaction. After completion of the addition of the bromine and the CDT, the reaction mixture was aged further at 30°C for 2 hours. After completion of the reaction, the post-treatment and the analysis were conducted in the same manner as in Examples 1 - 9. The results are summarized in Table 3.

Table 1

| | Example 1 | Example 2 | Example 3 | Example 4 | Example 5 |
|---|---|---|---|---|---|
| Reaction Solvent | Ethanol 124.6 ml | Ethanol 124.6 ml | Ethanol 124.6 ml | Ethanol 178.0 ml | n-Propanol 178.0 ml |
| | Dioxane 53.4 ml | Ethyl Acetate 53.4 ml | THF 53.4 ml | | |
| Bromine | 196.95 g (1.23 mol) | 196.95 g (1.23 mol) | 196.95 g (1.23 mol) | 196.95 g (1.23 mol) | 196.95 g (1.23 mol) |
| CDT | 50.00 g (0.31 mol) | 50.00 g (0.31 mol) | 50.00 g (0.31 mol) | 50.00 g (0.31 mol) | 50.00 g (0.31 mol) |
| Composition of Reaction Solution | | | | | |
| $\alpha$ - HBCD (wt%) | 18.19 | 19.62 | 17.36 | 19.77 | 15.85 |
| $\beta$ - HBCD (wt%) | 19.02 | 17.61 | 15.42 | 16.74 | 13.55 |
| $\gamma$ - HBCD (wt%) | 54.47 | 51.30 | 47.98 | 47.82 | 46.62 |
| $\alpha$ - TBCD (wt%) | 2.00 | 3.04 | 6.83 | 4.52 | 7.74 |
| $\beta$ - TBCD (wt%) | 4.62 | 5.64 | 9.67 | 7.92 | 11.28 |
| DBCD (wt%) | 0.23 | 0.51 | 0.50 | 0.00 | 2.86 |
| CDT (wt%) | 0.49 | 0.62 | 0.22 | 0.76 | 0.75 |
| Unknown (wt%) | 0.98 | 1.65 | 2.02 | 2.47 | 1.35 |
| Yield of HBCD (g) | 178.49 | 172.38 | 157.22 | 164.18 | 148.00 |
| Yield of HBCD (%) | 90.31 | 87.22 | 79.55 | 83.07 | 74.88 |
| Selectivity of $\gamma$ - HBCD (%) | 59.41 | 57.94 | 59.41 | 56.70 | 61.32 |
| Melting Point ($^{\circ}$C) | 188 ~ 191 | 185 ~ 190 | 183 ~ 187 | 183 ~ 186 | 182 ~ 185 |

Table 2

|  | Example 6 | Example 7 | Example 8 | Example 9 |
|---|---|---|---|---|
| Reaction Solvent | Ethanol 36.9 ml | Ethanol 71.2 ml | Ethanol 36.9 ml | Ethanol 71.2 ml |
|  | Methylene Chloride 30.2 ml | Methylene Chloride 106.8 ml | EDC 30.2 ml | EDC 106.8 ml |
| Bromine | 55.4 g (0.35 mol) | 197.0 g (1.23 mol) | 55.4 g (0.35 mol) | 197.0 g (1.23 mol) |
| CDT | 18.8 g (0.12 mol) | 50.0 g (0.12 mol) | 50.0 g (0.12 mol) | 50.0 g (0.12 mol) |
| Composition of Reaction Solution |  |  |  |  |
| $\alpha$ - HBCD (wt%) | 17.57 | 18.34 | 18.73 | 18.65 |
| $\beta$ - HBCD (wt%) | 20.15 | 20.08 | 20.48 | 20.51 |
| $\gamma$ - HBCD (wt%) | 53.21 | 53.08 | 52.90 | 52.40 |
| $\alpha$ - TBCD (wt%) | 2.59 | 2.91 | 2.90 | 2.50 |
| $\beta$ - TBCD (wt%) | 4.04 | 4.23 | 3.90 | 4.53 |
| DBCD (wt%) | 0.25 | 0.31 | 0.18 | 0.21 |
| CDT (wt%) | 0.00 | 0.00 | 0.00 | 0.00 |
| Unknown (wt%) | 1.01 | 1.05 | 2.89 | 1.20 |
| Yield of HBCD (g) | 67.85 | 179.78 | 66.40 | 179.50 |
| Yield of HBCD (%) | 91.52 | 90.38 | 89.56 | 90.24 |
| Selectivity of $\gamma$ - HBCD (%) | 58.52 | 58.01 | 57.43 | 57.23 |
| Melting Point (°C) | 190 ~ 193 | 189 ~ 193 | 190 ~ 192 | 190 ~ 192 |

Table 3

| | Comparative Example 1 | Comparative Example 2 | Comparative Example 3 | Comparative Example 4 |
|---|---|---|---|---|
| Reaction Solvent | Ethanol 124.6 ml | Ethanol 36.9 ml | Ethanol 36.9 ml | Ethanol 124.6 ml |
| | Dioxane 53.4 ml | Methylene Chloride 30.2 ml | EDC 30.2 ml | Dioxane 53.4 ml |
| Bromine | 196.95 g (1.23 mol) | 55.4 g (0.35 mol) | 55.4 g (0.35 mol) | 196.95 g (1.23 mol) |
| CDT | 50.0 g (0.31 mol) | 18.8 g (0.12 mol) | 18.8 g (0.12 mol) | 50.0 g (0.31 mol) |
| Composition of Reaction Solution | | | | |
| $\alpha$ - HBCD (wt%) | 18.04 | 13.79 | 13.36 | 18.26 |
| $\beta$ - HBCD (wt%) | 19.32 | 32.15 | 32.04 | 18.91 |
| $\gamma$ - HBCD (wt%) | 37.04 | 42.68 | 42.79 | 38.01 |
| $\alpha$ - TBCD (wt%) | 3.54 | 0.25 | 0.49 | 2.90 |
| $\beta$ - TBCD (wt%) | 8.24 | 0.32 | 0.94 | 5.42 |
| DBCD (wt%) | 0.56 | 0.00 | 0.00 | 0.72 |
| CDT (wt%) | 1.96 | 0.00 | 0.00 | 2.18 |
| Unknown (wt%) | 11.30 | 10.81 | 10.38 | 13.55 |
| Yield of HBCD (g) | 138.61 | 53.74 | 53.11 | 140.06 |
| Yield of HBCD (%) | 70.13 | 72.48 | 71.63 | 70.87 |
| Selectivity of $\gamma$ - HBCD (%) | 49.78 | 48.16 | 48.52 | 50.56 |
| Melting Point ($^\circ$C) | 165 ~ 170 | 166 ~ 170 | 165 ~ 171 | 166 ~ 172 |

Examples 10 - 24

A reaction solvent having a composition shown in Tables 4, 5, or 6 and bromine were placed in a round-bottomed flask equipped with a reflux condenser and a stirrer. Thereto, CDT in an amount shown in Tables 4, 5, or 6 was added dropwise at 30°C in 2 hours to cause reaction. After completion of the addition of the CDT, the reaction mixture was aged further at 30°C for 2 hours. The resulting slurry after the completion of the reaction was analyzed by high-performance liquid chromatography (column: TSK gel-ODS-80T made by Tosoh Corporation, eluent: acetonitrile/water = 80/20 vol ratio, wave length: UV 215 nm). The results of the analysis are shown collectively in Tables 4, 5, and 6. The components which could not be identified are shown as "unknown". In the Tables, DBCD (dibromocyclododecadiene) and TBCD (tetrabromocyclododecene) are reaction intermediates in the HBCD production. The TBCD includes isomers, which were analyzed by high-performance liquid chromatography using an ODS reversed phase column, and were named $\alpha$-TBCD and $\beta$-TBCD according to the order of elution from the chromatographic column.

7

The selectivity of $\gamma$-HBCD are represented by the ratio of the quantity of the yield of $\gamma$-HBCD to the total quantity of HBCD isomers ($\gamma$-HBCD / ($\alpha$-HBCD + $\beta$-HBCD + $\gamma$-HBCD)).

After completion of the reaction, the reaction mixture was filtered, and the separated crystalline matter was dried. The melting point thereof was measured. The results are shown collectively in Tables 4, 5, and 6.

Examples 25 - 32

A reaction solvent having a composition shown in Table 7 or Table 8, and CDT were placed in a round-bottomed flask equipped with a reflux condenser and a stirrer. Thereto, bromine in an amount shown in Table 7 or Table 8 was added dropwise at 30°C in 2 hours to cause reaction. After completion of the addition of the bromine, the reaction mixture was aged further at 30°C for 2 hours. After completion of the reaction, the post-treatment and the analysis were conducted in the same manner as in Examples 10 - 24. The results are summarized in Table 7 and Table 8.

Comparative Examples 5 - 8

A reaction solvent having a composition shown in Table 9, and bromine were placed in a round-bottomed flask equipped with a reflux condenser and a stirrer. Thereto, CDT in an amount shown in Table 9 was added dropwise at 30°C in 2 hours to cause reaction. After completion of the addition of the CDT, the reaction mixture was aged further at 30°C for 2 hours. After completion of the reaction, the post-treatment and the analysis were conducted in the same manner as in Examples 10 - 24. The results are summarized in Table 9.

Comparative Examples 9 - 12

A reaction solvent having a composition shown in Table 10, and CDT were placed in a round-bottomed flask equipped with a reflux condenser and a stirrer. Thereto, bromine in an amount shown in Table 10 was added dropwise at 30°C in 2 hours to cause reaction. After completion of the addition of the bromine, the reaction mixture was aged further at 30°C for 2 hours. After completion of the reaction, the post-treatment and the analysis were conducted in the same manner as in Examples 10 - 24. The results are summarized in Table 10.

Table 4

| | Example 10 | Example 11 | Example 12 | Example 13 | Example 14 |
|---|---|---|---|---|---|
| Reaction Solvent | Ethanol 540 ml | Ethanol 350 ml | Ethanol 216 ml | Ethanol 350 ml | Ethanol 350 ml |
| | Dioxane 230 ml | Dioxane 150 ml | Dioxane 92 ml | THF 150 ml | Ethyl Acetate 150 ml |
| Bromine | 61.6 g (0.39 mol) | 61.6 g (0.39 mol) | 61.6 g (0.39 mol) | 61.6 g (0.39 mol) | 61.6 g (0.39 mol) |
| CDT | 12.5 g (0.07 mol) | 12.5 g (0.07 mol) | 12.5 g (0.07 mol) | 12.5 g (0.07 mol) | 12.5 g (0.07 mol) |
| Substrate Concentration (CDT/Reaction Solvent)(wt/vol%) | 1.62 | 2.50 | 4.00 | 2.50 | 2.50 |
| Composition of Reaction Solution | | | | | |
| $\alpha$ - HBCD (wt%) | 13.13 | 13.75 | 14.23 | 12.36 | 12.42 |
| $\beta$ - HBCD (wt%) | 7.25 | 8.31 | 8.44 | 7.58 | 10.01 |
| $\gamma$ - HBCD (wt%) | 62.33 | 64.39 | 56.89 | 58.27 | 59.51 |
| $\alpha$ - TBCD (wt%) | 6.59 | 5.61 | 7.69 | 7.83 | 6.54 |
| $\beta$ - TBCD (wt%) | 9.59 | 6.89 | 11.53 | 10.67 | 9.14 |
| DBCD (wt%) | 0.14 | 0.00 | 0.23 | 0.22 | 0.66 |
| CDT (wt%) | 0.41 | 0.20 | 0.10 | 0.50 | 0.51 |
| Unknown (wt%) | 0.56 | 0.85 | 0.89 | 2.57 | 1.21 |
| Selectivity of $\gamma$ - HBCD (%) | 75.36 | 74.48 | 71.50 | 74.50 | 72.63 |
| Melting Point ($^{\circ}$C) | 195 ~ 198 | 196 ~ 197 | 195 ~ 198 | 192 ~ 196 | 193 ~ 195 |

Table 5

| | Example 15 | Example 16 | Example 17 | Example 18 | Example 19 |
|---|---|---|---|---|---|
| Reaction Solvent | Ethanol 626 ml | Ethanol 413 ml | Ethanol 206 ml | Ethanol 103 ml | Ethanol 52 ml |
| | Methylene Chloride 512 ml | Methylene Chloride 338 ml | Methylene Chloride 169 ml | Methylene Chloride 84 ml | Methylene Chloride 42 ml |
| Bromine | 92.3 g (0.58 mol) | 73.9 g (0.46 mol) | 69.2 g (0.43 mol) | 66.5 g (0.42 mol) | 64.6 g (0.40 mol) |
| CDT | 18.8 g (0.12 mol) | 18.8 g (0.12 mol) | 18.8 g (0.12 mol) | 18.8 g (0.12 mol) | 18.8 g (0.12 mol) |
| Substrate Concentration (CDT/Reaction Solvent)(wt/vol%) | 1.65 | 2.50 | 5.00 | 10.00 | 20.00 |
| Composition of Reaction Solution | | | | | |
| $\alpha$ - HBCD (wt%) | 8.30 | 9.06 | 10.30 | 10.60 | 10.50 |
| $\beta$ - HBCD (wt%) | 5.91 | 6.50 | 8.86 | 13.90 | 22.20 |
| $\gamma$ - HBCD (wt%) | 59.39 | 58.10 | 55.10 | 53.30 | 48.10 |
| $\alpha$ - TBCD (wt%) | 5.86 | 5.21 | 4.66 | 4.03 | 3.09 |
| $\beta$ - TBCD (wt%) | 7.98 | 7.70 | 7.55 | 7.02 | 6.45 |
| DBCD (wt%) | 10.60 | 10.40 | 11.60 | 9.75 | 7.51 |
| CDT (wt%) | 0.20 | 1.30 | 0.70 | 0.15 | 1.04 |
| Unknown (wt%) | 1.76 | 1.73 | 1.23 | 1.25 | 1.11 |
| Selectivity of $\gamma$ - HBCD (%) | 80.70 | 78.90 | 74.20 | 68.50 | 59.50 |
| Melting Point ($^\circ$C) | 196 ~ 198 | 195 ~ 198 | 196 ~ 198 | 196 ~ 198 | 196 ~ 198 |

Table 6

| | Example 20 | Example 21 | Example 22 | Example 23 | Example 24 |
|---|---|---|---|---|---|
| Reaction Solvent | Ethanol 626 ml | Ethanol 413 ml | Ethanol 206 ml | Ethanol 103 ml | Ethanol 52 ml |
| | EDC 512 ml | EDC 338 ml | EDC 169 ml | EDC 84 ml | EDC 42 ml |
| Bromine | 92.3 g (0.58 mol) | 92.3 g (0.58 mol) | 69.2 g (0.43 mol) | 66.5 g (0.42 mol) | 64.6 g (0.40 mol) |
| CDT | 18.8 g (0.12 mol) | 18.8 g (0.12 mol) | 18.8 g (0.12 mol) | 18.8 g (0.12 mol) | 18.8 g (0.12 mol) |
| Substrate Concentration (CDT/Reaction Solvent)(wt/vol%) | 1.65 | 2.50 | 5.00 | 10.00 | 20.00 |
| Composition of Reaction Solution | | | | | |
| $\alpha$ - HBCD (wt%) | 10.45 | 12.10 | 12.46 | 16.10 | 18.18 |
| $\beta$ - HBCD (wt%) | 8.15 | 8.54 | 9.86 | 15.32 | 16.15 |
| $\gamma$ - HBCD (wt%) | 69.98 | 68.90 | 63.21 | 59.13 | 57.21 |
| $\alpha$ - TBCD (wt%) | 4.15 | 3.85 | 4.70 | 2.51 | 2.05 |
| $\beta$ - TBCD (wt%) | 5.83 | 4.66 | 6.66 | 4.36 | 3.92 |
| DBCD (wt%) | 0.70 | 0.98 | 1.25 | 1.00 | 0.92 |
| CDT (wt%) | 0.30 | 0.10 | 0.62 | 0.53 | 0.65 |
| Unknown (wt%) | 0.44 | 0.92 | 1.24 | 1.05 | 0.92 |
| Selectivity of $\gamma$ - HBCD (%) | 79.00 | 76.95 | 73.90 | 65.30 | 62.50 |
| Melting Point ($^\circ$C) | 195 ~ 198 | 196 ~ 198 | 195 ~ 198 | 196 ~ 198 | 195 ~ 198 |

Table 7

| | Example 25 | Example 26 | Example 27 | Example 28 |
|---|---|---|---|---|
| Reaction Solvent | Ethanol 540 ml | Ethanol 175 ml | Ethanol 88 ml | Ethanol 125 ml |
| | Dioxane 230 ml | Dioxane 75 ml | Dioxane 38 ml | Dioxane 125 ml |
| Bromine | 37.0 g (0.23 mol) | 37.0 g (0.23 mol) | 37.0 g (0.23 mol) | 147.7 g (0.94 mol) |
| CDT | 12.5 g (0.07 mol) | 12.5 g (0.07 mol) | 12.5 g (0.07 mol) | 50.0 g (0.31 mol) |
| Substrate Concentration (CDT/Reaction Solvent)(wt/vol%) | 1.62 | 5.00 | 10.00 | 20.00 |
| Composition of Reaction Solution | | | | |
| $\alpha$ - HBCD (wt%) | 9.54 | 11.71 | 13.27 | 10.39 |
| $\beta$ - HBCD (wt%) | 8.01 | 13.65 | 13.94 | 13.39 |
| $\gamma$ - HBCD (wt%) | 46.69 | 44.66 | 44.64 | 38.92 |
| $\alpha$ - TBCD (wt%) | 12.91 | 9.32 | 8.00 | 9.57 |
| $\beta$ - TBCD (wt%) | 17.30 | 13.47 | 12.15 | 18.74 |
| DBCD (wt%) | 0.00 | 0.74 | 1.43 | 0.97 |
| CDT (wt%) | 1.99 | 2.45 | 2.55 | 2.17 |
| Unknown (wt%) | 3.56 | 4.00 | 4.02 | 4.85 |
| Selectivity of $\gamma$ - HBCD (%) | 72.68 | 63.78 | 62.13 | 61.10 |
| Melting Point (°C) | 196 ~ 198 | 192 ~ 197 | 188 ~ 192 | 189 ~ 191 |

Table 8

| | Example 29 | Example 30 | Example 31 | Example 32 |
|---|---|---|---|---|
| Reaction Solvent | Ethanol 300 ml | Ethanol 103 ml | Ethanol 300 ml | Ethanol 103 ml |
| | Methylene Chloride 450 ml | Methylene Chloride 84 ml | EDC 450 ml | EDC 84 ml |
| Bromine | 55.4 g (0.35 mol) | 55.4 g (0.35 mol) | 55.4 g (0.35 mol) | 55.4 g (0.35 mol) |
| CDT | 18.8 g (0.12 mol) | 18.8 g (0.12 mol) | 18.8 g (0.12 mol) | 18.8 g (0.12 mol) |
| Substrate Concentration (CDT/Reaction Solvent)(wt/vol%) | 2.50 | 10.00 | 2.50 | 10.00 |
| Composition of Reaction Solution | | | | |
| $\alpha$ - HBCD (wt%) | 10.50 | 10.95 | 12.83 | 17.38 |
| $\beta$ - HBCD (wt%) | 12.00 | 17.16 | 12.13 | 15.32 |
| $\gamma$ - HBCD (wt%) | 55.00 | 50.26 | 58.25 | 55.92 |
| $\alpha$ - TBCD (wt%) | 5.12 | 5.01 | 3.52 | 1.10 |
| $\beta$ - TBCD (wt%) | 9.68 | 9.05 | 7.15 | 2.80 |
| DBCD (wt%) | 2.00 | 1.98 | 1.25 | 1.05 |
| CDT (wt%) | 0.53 | 0.02 | 0.82 | 0.35 |
| Unknown (wt%) | 5.17 | 5.57 | 4.05 | 6.08 |
| Selectivity of $\gamma$ - HBCD (%) | 71.00 | 64.13 | 70.00 | 63.10 |
| Melting Point (°C) | 195 ~ 198 | 192 ~ 197 | 195 ~ 198 | 192 ~ 197 |

EP 0 429 059 A2

Table 9

| | Comparative Example 5 | Comparative Example 6 | Comparative Example 7 | Comparative Example 8 |
|---|---|---|---|---|
| Reaction Solvent | Ethanol 124.6 ml | Ethanol 178.0 ml | Ethanol 36.9 ml | Ethanol 36.9 ml |
| | Dioxane 53.4 ml | | Methylene Chloride 30.2 ml | EDC 30.2 ml |
| Bromine | 197.0 g (1.23 mol) | 197.0 g (1.23 mol) | 55.4 g (0.35 mol) | 55.4 g (0.35 mol) |
| CDT | 50.0 g (0.31 mol) | 50.0 g (1.23 mol) | 18.8 g (0.12 mol) | 18.8 g (0.12 mol) |
| Substrate Concentration (CDT/Reaction Solvent)(wt/vol%) | 28.1 | 28.1 | 28.1 | 28.1 |
| Composition of Reaction Solution | | | | |
| $\alpha$ - HBCD (wt%) | 18.19 | 19.77 | 17.57 | 18.73 |
| $\beta$ - HBCD (wt%) | 19.02 | 16.74 | 20.15 | 20.48 |
| $\gamma$ - HBCD (wt%) | 54.47 | 47.82 | 53.21 | 52.90 |
| $\alpha$ - TBCD (wt%) | 2.00 | 4.52 | 2.59 | 2.90 |
| $\beta$ - TBCD (wt%) | 4.62 | 7.92 | 4.04 | 3.90 |
| DBCD (wt%) | 0.23 | 0.00 | 0.25 | 0.18 |
| CDT (wt%) | 0.49 | 0.76 | 0.00 | 0.00 |
| Unknown (wt%) | 0.98 | 2.47 | 1.01 | 0.89 |
| Selectivity of $\gamma$ - HBCD (%) | 59.41 | 56.71 | 58.52 | 57.43 |
| Melting Point ($^\circ$C) | 188 ~ 191 | 183 ~ 186 | 186 ~ 190 | 184 ~ 189 |

14

EP 0 429 059 A2

Table 10

| | Comparative Example 9 | Comparative Example 10 | Comparative Example 11 | Comparative Example 12 |
|---|---|---|---|---|
| Reaction Solvent | Ethanol 124.6 ml | Ethanol 62.5 ml | Ethanol 36.9 ml | Ethanol 36.9 ml |
| | Dioxane 53.4 ml | Dioxane 62.5 ml | Methylene Chloride 30.2 ml | EDC 30.2 ml |
| Bromine | 197.0 g (1.23 mol) | 147.7 g (0.94 mol) | 55.4 g (0.35 mol) | 55.4 g (0.35 mol) |
| CDT | 50.0 g (0.31 mol) | 50.0 g (0.31 mol) | 18.8 g (0.12 mol) | 18.8 g (0.12 mol) |
| Substrate Concentration (CDT/Reaction Solvent)(wt/vol%) | 28.1 | 40.0 | 28.1 | 28.1 |
| Composition of Reaction Solution | | | | |
| $\alpha$ - HBCD (wt%) | 18.04 | 17.96 | 13.79 | 13.36 |
| $\beta$ - HBCD (wt%) | 19.32 | 25.00 | 32.15 | 32.04 |
| $\gamma$ - HBCD (wt%) | 37.04 | 35.15 | 42.68 | 42.79 |
| $\alpha$ - TBCD (wt%) | 3.54 | 2.58 | 0.25 | 0.49 |
| $\beta$ - TBCD (wt%) | 8.24 | 5.59 | 0.32 | 0.94 |
| DBCD (wt%) | 0.56 | 0.00 | 0.00 | 0.00 |
| CDT (wt%) | 1.96 | 0.00 | 0.00 | 0.00 |
| Unknown (wt%) | 11.30 | 13.72 | 10.81 | 10.38 |
| Selectivity of $\gamma$ - HBCD (%) | 49.78 | 45.00 | 48.16 | 48.52 |
| Melting Point ($^\circ$C) | 165 ~ 170 | 165 ~ 169 | 185 ~ 189 | 186 ~ 190 |

## Claims

1. A process for producing 1,2,5,6,9,10-hexabromocyclododecane by reacting bromine with 1,5,9-cis,trans,trans-cyclododecatriene in the presence of an organic solvent, the process comprising adding dropwise 1,5,9-cis,trans,trans-cyclododecatriene to a solution of bromine in an alcohol of 1 - 4 carbons or in an organic solvent containing the alcohol.

2. A process for producing 1,2,5,6,9,10-hexabromocyclododecane by reacting bromine with 1,5,9-cis,trans,trans-cyclododecatriene in the presence of an alcohol of 1 - 4 carbons or an organic solvent containing the alcohol as a reaction solvent, the concentration of the 1,5,9-cis,trans,trans-cyclododecatriene substrate being in the range of from 0.1 to 20 weight/volume % relative to the reaction solvent.

3. A process according to claim 1 wherein the organic solvent is selected from the group consisting of ethers, halogenated hydrocarbons, esters.

4. A process according to claim 1 wherein the organic solvent containing the alcohol is selected from the group consisting of ethanol-ethyl acetate, ethanol-THF, ethanol-dioxane, ethanol-methylene chloride, and ethanol-EDC.

5. A process according to claim 1 wherein the reaction is performed in a temperature range from about -20$^\circ$ to 50$^\circ$ C.

15

6. A process according to claim 1 wherein the amount of bromine relative to CDT is not less than 3.0 in terms of $Br_2$/CDT (molar ratio).